# EUROPEAN PATENT APPLICATION

(11) **EP 2 815 654 A1**
(43) Date of publication of application: **24.12.2014**
(21) Application number: 13173297.6
(22) Date of filing: 21.06.2013
(51) Int. Cl.: A23L 1/302, A61K 31/01, A61K 31/366, A61K 31/593, A61K 36/06, A61P 9/00

(54) **Composition having a beneficial effect on the cardiovascular system, comprising monacolin K, lycopene and vitamin D3**

(71) Applicant: Essential IP BV, 1404 AK Bussum (NL)
(72) Inventor: Eijgelaar, Wouter-Jan, 3824 VP Amersfoort (NL); Schoevers, Peter Alexander, 1404 AK Bussum (NL); Viëtor, Hendrik Engelbertus, 1404 AK Bussum (NL)
(74) Representative: Habets, Winand

(57) **Abstract**

The present invention is in the field of medical treatment and relates to a formulation for oral administration, such as for instance in the form of tablets or of a powder, which is able to exert a beneficial effect on the cardiovascular system. More in particular, the invention provides a composition for oral administration having a beneficial effect on the cardiovascular system, comprising as active ingredients a mixture of monacolin K, lycopene and vitamin D3.

## Description

### Field of the invention

The present invention is in the field of medical treatment and relates to a formulation for oral administration, such as for instance in the form of tablets or of a powder, which is able to exert a beneficial effect on the cardiovascular system.

### Background of the invention

In the industrialized countries, cardiovascular diseases are among the main causes of death for men and women [Schwartz LM, Woloshin S: Changing disease definitions: implications for disease prevalence, Analysis of the Third National Health and Nutrition Examination Survey, 1988-1994. Eff Clin Pract 1999; 2:76-85, Lowe LP, Greenland P, Ruth KJ et al.: Impact of major cardiovascular disease risk factors, particularly in combination, on 22-year mortality in women and men. Arch Intern Med 1998; 158:2007-2014, Sempos CT, Ceeman JI, Carroll MD et al.: Prevalence of high blood cholesterol among US adults. An update based on guidelines from the second report of the National Cholesterol Education Program Adult Treatment Panel. JAMA 1993; 269:3009-3014].

Many cardiovascular diseases are of an atherosclerotic nature, i.e. are due to narrowing of the lumen of the arteries owing to the deposition of cholesterol, calcium and fibrin in the vessel wall, or to occlusion of the arteries caused by a thrombus that formed from an atheromatous plaque. As was documented in the Framingham epidemiological study [Kannel WB, Castelli WP, Gordon T et al. Lipoprotein cholesterol in the prediction of atherosclerotic disease: new perspectives based on the Framingham Heart Study. Ann Intern Med 1995; 90:85-91], the incidence and the severity of atherosclerosis are closely related to the cholesterol level in the blood.

It is therefore very important to keep blood cholesterol under control. Another important cardiovascular risk factor is represented by increased levels of triglycerides (above 150-200 mg/dl), especially if accompanied by a reduced HDL cholesterol level (< 40 mg/dl) or within the so-called "metabolic syndrome". It is therefore also important to lower the level of triglycerides in the blood. Dietary supplements may help to achieve that goal.

Another pathogenetic factor of atherosclerosis is peroxidation of LDL. In fact LDLs are transported in the blood without particular pathological problems. However, if they are peroxidized by ROS (Reactive Oxygen Species); they pass through the endothelium of the arteries and are captured and stored by macrophages. The latter, packed with peroxidized LDL, are transformed to so-called "foam cells", which represent the initial nucleus of atheromas of the intima, from which atherosclerotic plaques then form. Foam cell formation is considered one of the main drivers of atheromatous plaque formation and progression of atherosclerotic disease.

Antioxidants that inhibit the peroxidation of LDL may therefore be very useful for preventing atherosclerosis. This objective can be achieved with an appropriate choice of foods and with the support of suitable dietary supplements rich in antioxidants.

Suitable dietary supplements may therefore be advantageously employed for controlling the cholesterol level in the blood when this is slightly or moderately raised. Dietary supplements may be particularly useful for subjects with "intermediate" overall risk, or those having a probability between 10% and 20% of developing a cardiovascular disease in the next 10 years of life, but for whom therapy with cholesterol-lowering drugs is not yet justified.

There are at present various dietary supplements that can provide beneficial action and give a significant reduction of cardiovascular risk, especially if combined with a controlled diet and with healthy physical activity. There remains a need however for more effective, more efficient and safer compositions for improving the health or condition of the vascular system. In particular there is a need for such compositions based on natural ingredients as opposed to synthetic ingredients.

### Summary of the invention

The invention relates to a composition for oral administration having a beneficial effect on the cardiovascular system, comprising as active ingredients a mixture of monacolin K, lycopene and vitamin D3. Such a composition may further advantageously comprise berberine, coenzyme Q10, panax ginseng, flavenoids, vitamin K2, folic acid, vitamin B, biotin or minerals.

The invention also relates to a composition as described above for use in the treatment or prevention of a disease or for reducing foam cell formation and progression. A particular preferred use of the composition according to the invention is in the treatment of heart disease, atherosclerose, cardiovascular disease, hypercholesterolaemia, hypertriglyceridaemia, and other diseases related to an increased uptake of oxLDL by foam cells.

### Detailed description of the invention

Atherosclerosis and the resulting plaque formation in the vessel wall is the underlying mechanism of cardiovascular disease. Atherosclerosis is a progressive inflammation driven process. It is believed that disturbed cholesterol levels, hypertension and elevated blood glucose levels not only contribute to disease progression, but are also driven by the same inflammatory processes that cause atherosclerosis.

We employed a model for measuring the oxLDL uptake by macrophages, a known and accepted model for determining foam cell formation, which in turn is an accepted measure for plaque formation as well as for the development of atherosclerosis and decreasing vascular health in general. Our experimental data are therefore relevant for determining the beneficial effects of several active ingredients on the cardiovascular system.

More in detail, we stimulated human human monocytic THP-1 cells [Tsuchiya S, Yamabe M, Yamaguchi Y, Kobayashi Y, Konno T, Tada K (August 1980). "Establishment and characterization of a human acute monocytic leukemia cell line (THP-1)". Int. J. Cancer 26 (2): 171-6. doi:10.1002/ijc.2910260208.] with phorbol 12-myristate 13-acetate (PMA) and contacted or pre-treated the cells with a number of active ingredients. After 24 hours of pre-treatment, the cells were contacted with or exposed to fluorescently labeled oxLDL and uptake of oxLDL was determined after 24 hours (see example 1).

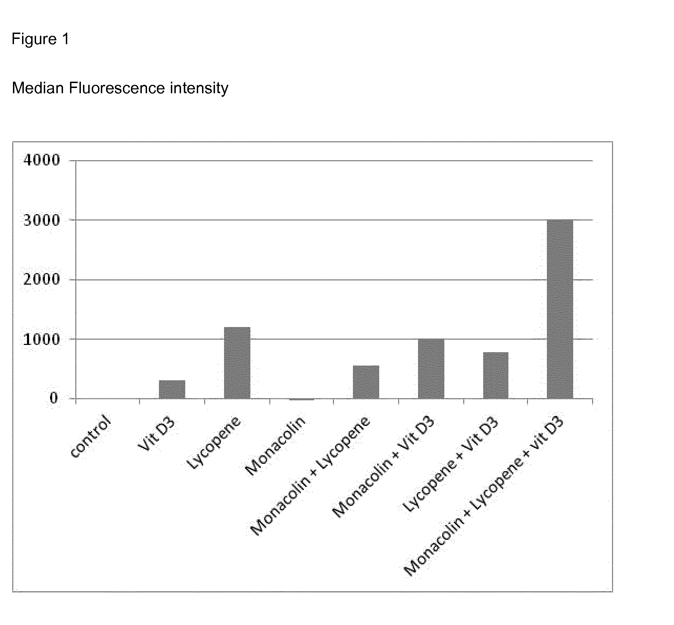

We found that addition of vitamin D3 and monacolin K had no effect on the uptake of oxLDL, whereas lycopene slightly inhibited the uptake of oxLDL (figure 1).Compositions comprising a combination of these three active ingredients did not alter the inhibitory effects significantly. Surprisingly, a composition comprising all 3 active ingredients exhibited a synergistic effect. When monacolin K, vitamin D3 and lycopene were combined in one composition, that composition gave rise to an inhibition of the uptake of oxLDL that was far greater than the sum of the effects of the active ingredients individually (example 1, figure 1).

Uptake of oxLDL by macrophages leads to the development of foam cells, which contribute to atherosclerotic plaque formation and progression. The invention therefore relates to a composition having a beneficial effect on the cardiovascular system, comprising as active ingredients a mixture of monacolin K, lycopene and vitamin D3. Such a composition is preferably for use in oral administration.

Whereas the individual active ingredients are known and have been described for various medicinal use, a composition as described above is novel. Moreover, the composition according to the invention surprisingly showed a synergistic effect on the inhibition of oxLDL uptake of macrophages, thereby reducing the formation of foam cells. This has a beneficial effect on the cardiovascular system and reduces the chances of acute myocardial infarction and stroke, as well as atherosclerotic plaque formation. A composition according to the invention provides the additional advantage that it may contain less of each of the active ingredients than in a comparable composition that comprises each of the ingredients separately, whereas it achieves the same effect. This feature allows for the addition of other ingredients, since the number of ingredients that is contained in a composition is limited. This is attributable to the fact that the weight of a unit dose for oral administration may be approximately 1 gram in total. Above that weight, the unit dose becomes difficult to swallow, a unit dose of 5 grams is almost impossible to swallow. The fact that the three ingredients mentioned above synergistically improve their effect, allows for further additional active ingredients to be contained in the same unit dose.

A composition according to the invention also has a strong anti-inflammatory effect. As such, a composition according to the invention may significantly contribute to the prevention of cardiovascular disease (CVD), by lowering the CVD risk factors and reducing inflammation. Therefore, the composition according to the invention has the potential to reduce disease progression by early intervention, delaying or preventing the need for medical treatment of hypercholesterolemia, hypertension and type 2 diabetes.

The individual active ingredients of the composition according to the invention are herein described in detail.

"A", "an", and "the" as used herein refers to both singular and plural referents unless the context clearly dictates otherwise. By way of example, "a compartment" may refer to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.

The recitation of numerical ranges by endpoints includes all numbers and fractions subsumed within that range, as well as the recited endpoints.

The expression "percentage" "%" or "% by weight" (weight percent), here and throughout the description unless otherwise defined, refers to the relative weight of the respective component based on the overall weight of the formulation.

Monacolin K is a major constituent of red yeast rice [references 2 - 13]. Red Yeast Rice is rice that has been fermented with the yeast Monascus Purpureus. Its constituent called monacolin K has similar properties as the commonly known statins in cholesterol lowering medicine. Monacolin K is also known as the statin Lovastatin or Mevacor, which is marketed by Merck as cholesterol lowering medicine. It lowers the body's cholesterol production by inhibiting HMG-CoA reductase in the liver.

Over 100 clinical trials have been performed that analyzed the cholesterol lowering properties and preventive efficacy of Red Yeast Rice. These studies all show that daily consumption of Red Yeast Rice has similar cholesterol lowering properties as medicinal statins, but with fewer side effects. A meta-analysis of 9625 patients in 93 randomized trials involving 3 different commercial variants of Red Yeast Rice has summarized this large experience (4). In addition, a standardized Red Yeast Rice extract called Xuezhikang was evaluated in a large, randomized, placebo-controlled clinical trial, the China Coronary Secondary Prevention Study (CCSPS). With a duration of 4.5 years the CCSPS enrolled 4870 participants (3986 men, 884 women) with a previous myocardial infarction and showed significant reduction in the primary endpoint, nonfatal myocardial infarction or death from coronary or cardiac causes. The secondary endpoints, including total mortality from cardiovascular disease, total all-cause mortality, need for coronary revascularization procedure, and change in lipid levels, were also significantly reduced, with the exception of fatal myocardial infarction. The number needed to treat (NNT) to prevent a primary end point over the 4.5-year duration of the trial is 21, which favorably compares with the NNT range (19-56) observed in previous secondary prevention studies of statins.

Scientific evidence shows that daily intake of Red Yeast Rice containing 10mg monacolin K results in LDL cholesterol lowering of 7-25%, relative increase of HDL up to 17% and lowering of triglyceride blood levels by 10-44%. By lowering these risk factors the cardiovascular risk was reduced by 30-50% (2-13).

Besides cholesterol lowering, statins also have anti-inflammatory properties. This is a major constituent of the preventive effects of statins on cardiovascular disease. Several meta-analyses together containing over 30 clinical studies show that statin use reduces the blood levels of a number of well-known inflammatory markers (70, 71). In addition, statins have been shown to have immunomodulator effects in immune diseases like rheumatoid arthritis (72). Meta-analyses of over 20 studies show that stains help to prevent infections and infection induced mortality (73, 74). Recent studies show that daily statin use in low-risk individuals results in a significant reduction of cardiovascular risk and cardiovascular related mortality (75).

Besides natural statins, Red Yeast Rice also contains a series of compounds with beneficiary effects on cardiovascular disease, such as polyunsaturated fatty acids, ergosterol, amino acids, flavonoids, alkaloids, tannins and different trace elements and minerals (76).

The safety of Red yeast Rice consumption has been elaborately shown in recent clinical trials and meta-analyses. In the large meta-analysis that evaluated 93 randomized clinical trials (including 9625 participants), Red Yeast Rice preparations showed cholesterol-lowering effects similar to those of low-dose statins with no relevant adverse effects (4). Red Yeast Rice extract with a 0.2 % monacolin K content is well tolerated, also in statin intolerant subjects, at the dosage of 0.6, 1.2, or 2.4 g twice daily, (90, 91). A study that specifically investigated the side effects of 1.8 g/day Red Yeast Rice in patients that did not tolerate standard statin therapy, showed that 93 % of subjects with a history of statin-associated myalgia were able to tolerate 3.6 grams (equivalent to 6mg lovastatin) of Red Yeast Rice daily for 24 weeks without a recurrence of myalgia. Furthermore, in this study Red Yeast Rice did not increase the mean brief pain inventory (BPI) score, which represents reported average pain over the past month on a scale of 1-10. There also was no significant difference in creatine phosphokinase, aspartate aminotrasferase, or alanine aminotransferase levels between treatment and control groups. In this study 56% of subjects achieved target cholesterol levels (91). A one year study of a dietary supplement containing Berberine 500 mg, Policosanol 10 mg, Red Yeast Rice 200 mg, Folic acid 0.2 mg, Coenzyme Q10 2.0 mg, and Astaxanthin 0.5 mg in 80 elderly hypercholesterolemic patients who were previously statin-intolerant, showed that acceptable plasma LDL-C levels can be achieved without significant side-effects or safety issues. In addition, no significant differences in creatine phosphokinase, aspartate aminotrasferase, or alanine aminotransferase levels between treatment and control groups were reported (19). Interestingly however, there are also a number of case reports in the literature of muscle myopathy and liver damage resulting from red yeast rice usage (82-85, 92-94).

In conclusion, at comparable doses, the monacolin K efficacy and safety profile is similar to that of statins. In a composition according to the invention, monacolin K may preferably be present in the range of 2 - 40 mg per unit dose.

A unit dose is defined herein as a single-unit container so designed that the contents are administered to the patient as a single dose, direct from the container. Preferably, the unit dose comprises the daily dose so that a patient only has to take a single unit per day. A unit dose may comprise a pill, sachet, capsule, bulk powder container, tablet or equivalent suited for oral administration to a subject, preferably a human.

In a preferred composition according to the invention a low dose of citrinin free Red Yeast Rice extract containing 10 mg monacolin K per dose unit is used. This is 2 times less than the commonly used 20 mg starting dose, and 40-100 mg treatment dose of Lovastatin, thereby reducing the risk of side-effects.

Lycopene (from the New Latin word lycopersicum for the tomato species name) is a bright red carotenoid pigment and phytochemical found in tomatoes and other red fruits and vegetables, such as red carrots, red bell peppers, watermelons, and papayas (but not strawberries or cherries). Lycopene is not an essential nutrient for humans, but is commonly found in the diet, mainly from dishes prepared from tomatoes. Lycopene has been found to possess antioxidant and antiproliferative properties in animal and laboratory studies, although activity in humans is not yet adequately determined. Lycopene is currently marketed as a protective agent against prostate cancer. However, conclusive evidence for this effect is lacking. Lycopene does have positive effects on cholesterol levels and blood pressure, potentially reducing cardiovascular disease risk.

To our knowledge, no case control studies that evaluated Lycopene supplementation and the risk of cardiovascular disease are available. There are, however, several studies that associated high dietary lycopene consumption with cardiovascular disease risk. In a major European study, men who ate large amounts of lycopene-rich foods were 50% less likely to have a heart attack than men who consumed little lycopene. Nonsmokers experienced the most benefit (113). Some epidemiological research also suggests that women with higher serum lycopene levels have a reduced risk of developing heart disease (114). However, conflicting evidence exists. A seven-year prospective cohort study of 39,876 middle-aged and older women assessed whether intake of lycopene or tomato-based foods is associated with cardiovascular disease prevention. Results from the study showed no association between dietary lycopene intake and the risk of heart attack, stroke, and other cardiovascular events in women (115). The dietary intake in these studies is significantly lower than what is achieved by lycopene supplementation. In addition high dietary lycopene intake often is associated with other dietary differences, making it hard to draw definite conclusions.

In a meta-analysis of 12 studies that investigated the effect of lycopene consumption on serum lipids, and four studies that examined its effect on blood pressure including data of 694 participants, revealed a significant cholesterol-lowering effect of lycopene for total serum cholesterol and LDL cholesterol by 10%, while HDL levels remained unchanged. This result was only achieved in the subgroup of trials using lycopene dosages of ≥25mg daily, whereas subgroup meta-analysis of trials using lower lycopene dosages was not significant (28). Another study showed that lycopene intervention reduces inflammation and improves HDL functionality in moderately overweight middle-aged individuals (29). The protective effect of Lycopene can also be deducted from a study in 1031 Finnish men aged 46-65 years of the Kuopio Ischemic Heart Disease Risk Factor (KIHD) cohort that showed that low blood levels of lycopene and/or β-carotene are associated with increased risk of sudden cardiac death (25-27). A recent study showed that lycopene inhibits 3-hydroxy-3-methylglutaryl coenzyme A (HmG-CoA) reductase in white blood cells, specifically, monocytes and macrophages, reducing or preventing foam cell formation. Foam cell formation is an important aspect of cardiovascular disease development. This inhibitory effect on HMG-CoA reductase was confirmed in another study (116). This mechanism of action is similar to that of statins (117).

A relatively small study in 25 subjects indicated that consuming Lycopene rich tomato products with a high-fat meal attenuates postprandial lipemiainduced oxidative stress and associated inflammatory response. The relevance of oxidized LDL and inflammation to vascular injury suggests a potentially important protective role of lycopene in reducing cardiovascular disease risk (118).

A meta-analysis of a limited number of 4 studies to the effect of lycopene on systolic blood pressure suggested a significant blood pressure reducing effect of 5.60 mmHg. Blood pressure reduction was more pronounced (9,35 mmHg) when a subgroup of trials with high blood pressure at baseline (SBP ≥ 140 mm Hg) was analyzed (28).

In one of the largest, most comprehensive studies evaluating prostate cancer risk and lycopene, a cohort of more than 47,800 men completed a 131-item questionnaire in 1986 and every few years after. Men with higher estimated lycopene content in their diets had a lower risk of developing prostate cancer. A risk reduction of 35% was observed for men who consumed ten or more servings of tomato products per week compared to those with fewer than 1.5 servings per week. Interestingly, tomato sauce accounted for the strongest reduction in risk for any one specific food item (119). But a 2004 meta-analysis of 11 studies found that while tomato products play a role in preventing prostate cancer, their role is modest and requires high amounts of tomato intake (120). A recent Cochrane meta-analysis on the protective effects against prostate cancer remains inconclusive (121-123). Three randomized controlled clinical trials, with a total of 154 participants were included in this review. None of the studies reported data on prostate cancer mortality. All of the included studies differed with respect to design, participants included and allocation of lycopene. This clinical heterogeneity and the reported 'high' risk of bias limits the value of the meta-analyses. Meta-analysis indicated no statistical difference in PSA levels between lycopene and placebo. PSA is a biomarker for prostate cancer. Only one study reported a decreased incidence of prostate cancer, 10% in the lycopene group versus 30% in control group. Another meta-analysis showed no correlation between lycopene consumption and breast cancer risk (20). The role of lycopene supplementation is not yet clear. Preliminary evidence shows that in men with existing high-grade prostate intraepithelial neoplasia (pre-malignant cellular changes seen in biopsy specimens), taking 4 mg of lycopene twice daily may delay or prevent progression to prostate cancer (125). However, preliminary evidence indicates lycopene may worsen established prostate cancer by increasing metastasis without having any effect on cancer cell proliferation (126). In all there is insufficient evidence to either support, or refute the use of lycopene for the prevention of prostate cancer. Similarly, there is no robust evidence from clinical trials to identify the impact of lycopene consumption upon the incidence of prostate cancer, prostate symptoms, PSA levels or adverse events.

In a 2005 prospective cohort study of dietary lycopene and its food sources evaluated in 39,876 women, neither higher dietary nor plasma lycopene levels were associated with a reduced risk of breast cancer in middle-aged and older women (127). In the 2007 evidence-based review by the FDA, data showed limited evidence to support an association between lycopene intake or tomato consumption with a reduced risk of breast cancer (128). Blood levels of lycopene and other carotenoids do, however, show significant associations with reduce breast cancer risk (129). However, some positive relationships between lycopene and reduced risk of breast cancer were shown in the Women's Health Initiative studies and in the Shanghai Women's Health Study when subgroups of carotenoids were examined, but these effects have not been examined individually in clinical trials (130, 131). In a 2007 evidence-based review, the U.S. Food and Drug Administration (FDA) found only limited evidence to support an association between lycopene intake or tomato consumption with a reduced risk of gastric cancer (128).

Lycopene, extracted from tomatoes or other natural sources is nontoxic, is generally considered safe and is authorised as a food colouring agent within the EU (E160d) (Directive 94/36/EC) and the US (CDR 21 73.295). It is a common food ingredient. An overview of average dietary intakes of lycopene from foods in different populations was presented in previous EFSA evaluations. Regular intakes of lycopene from natural dietary sources are estimated to be on average between 0.5 and 5 mg/day, with high exposures up to about 8 mg/day. High consumption of fruits and vegetables, especially tomato products, may result in occasional intakes of 20 mg/day or more. The acceptable daily intake (ADI) established by the Scientific Panel on food additives, flavourings, processing aids and materials in contact with food (AFC Panel) is 0.5 mg/kg/day for lycopene from all sources (EFSA, 2008). This translates to a daily intake of 30 mg Lycopene for someone weighing 60 kg. The dose of 25 mg in a preferred composition according to the invention lies well within this ADI.

There are no known serious side effects, but the safety of long-term intake of high doses of lycopene supplements has not been thoroughly studied. Review of available scientific literature finds tomatoes, tomato-based products, and lycopene supplements generally well tolerated. Daily supplements containing 30 mg of lycopene have been used safely for up to 8 weeks However, rare reports of diarrhea, nausea, stomach pain or cramps, gas, vomiting, and loss of appetite have been reported by some individuals who took a lycopene-rich tomato supplement of 15 milligrams twice a day. There are no known interactions between lycopene and existing drugs.

One important, but still unclear, safety concern is the fact that preliminary evidence indicates lycopene may worsen established prostate cancer by increasing metastasis without having an effect on cancer cell proliferation. Therefore, men with established prostate cancer should avoid using lycopene until more is known (126).

A composition according to the invention may contain between 1 and 100 mg lycopene per unit dose, such as between 5 and 50 mg. Preferred is a dose of 25 mg per unit dose.

Recent research established that a wide range of health benefits that can be achieved by vitamin D supplementation. The best known are the muscular and the skeletal benefits to prevent Rickets. The Mayo Clinic provides a very good overview of existing scientific evidence for the health benefits of vitamin D supplements at http://www.mayoclinic.com/health/vitamin-d/NS_patient-vitamind/DSECTION=evidence the content of which is incorporated by reference herein.

The current International Osteoporosis Guidelines determine vitamin D insufficiency as vitamin D (25-OHD) blood levels <50 nmol/L, while vitamin D deficiency is determined as 25-OHD blood levels <25 nmol/L. Based on these Guidelines the majority of people (aged >45 years) has insufficient or sub-optimal vitamin D levels (187-189). The summary below will focus on the benefits of vitamin D supplementation in cardiovascular disease prevention.

Vitamin D deficiency or sub-optimal vitamin D levels are related with increased cardiovascular risk. Vitamin D deficiency has been linked with hypertension, immune function, myocardial infarction, stroke, and other cardiovascular-related diseases including atherosclerosis and endothelial dysfunction (45-49). A large prospective, case-controlled study of 18,000 men showed a significant correlation between low vitamin D levels and an increased risk for myocardial infarction (50). A Meta-analysis of 24 studies containing a total of 6123 cardiovascular disease cases in 65 994 participants, also showed that lower vitamin D levels are linearly associated with increased cardiovascular risk and increased risk of cardiovascular disease-related mortality (51-55).

A meta-analysis of 17 prospective cohort studies and randomized trials found moderate to high doses of vitamin D supplementation may decrease the risk for cardiovascular disease. However, the amount of studies are limited and the available data is not very consistent (190). Only one study in the general population showed a very clear decrease in cardiovascular disease after vitamin D supplementation (56). In another trial of 148 women supplementation with vitamin D and calcium resulted in a significant increase in vitamin D blood levels by 72% and significant decreases in systolic blood pressure and heart rate compared with calcium supplementation alone (57). However, a different meta-analysis of 51 trials examining the effects of vitamin D supplements on cardiovascular outcomes found that although some trials showed benefits of vitamin D supplementation on risk factors, the overall trial data available could not demonstrate a consistent and statistically significant reduction in mortality or cardiovascular risk after vitamin D supplementation (191, 192). The authors do suggest a positive effect of vitamin D supplements as was seen in a different meta-analysis (55). They blame the fact that many of the included studies were not designed to evaluate cardiovascular outcomes.

Recent studies show low vitamin D levels are associated with hypertension and left ventricular hypertrophy. Supplementation with vitamin D provides relieve for left ventricular atrophy (58, 59). Two meta-analyses have looked at the effect of vitamin D on blood pressure levels. These meta-analyses did not demonstrate a significant effect of vitamin D on blood pressure (193, 194). However, included trials were small and significant heterogeneity was observed in both meta-analyses. Better designed trials are needed to thoroughly investigate the benefits of vitamin D supplementation in lowering blood pressure.

Other studies showed an association between low vitamin D levels and endothelial dysfunction and arterial stiffness (49). Supplementation of vitamin D in patients with low vitamin D levels showed significant improvement in arterial stiffness when compared with placebo. Furthermore studies have also shown an association between vitamin D supplementation and a decrease in pulse wave velocity and arterial stiffness (60, 61) and an inverse association between vitamin D levels and subclinical atherosclerosis as measured by carotid intimal-media thickness (62). These studies do suggest a beneficial effect of vitamin D supplementation on cardiovascular risk.

Studies show a chronic deficiency in vitamin D among people aged over 45, this is mostly acclaimed to the lack of exposure to sunlight and reduced vitamin D production. In addition, research shows that more and more age related disorders are associated to sub-optimal vitamin D levels. Although conclusive evidence of the health benefits of vitamin D supplements on cardiovascular disease risk is still lacking, many studies suggest positive effects. Despite the limited evidence for cardiovascular disease prevention, the recommendation of vitamin D supplements is justified by the wide spread insufficiency and the scientifically proven muscular and the skeletal health benefits. In conclusion, vitamin D supplements are recommended for people >45 years of age.

A composition according to the invention preferably comprises between 1 and 100 microgram of vitamin D, more preferably between 5 and 50, such as 25 microgram per unit dose.

In addition to the above described active ingredients, a composition according to the invention may also contain a number of additional ingredients. The invention therefore also relates to a composition as described herein further comprising an active ingredient selected from the group consisting of berberin, coenzyme Q10, panax Ginseng, flavenoids, vitamin K2, folic acid, vitamin B, biotin and minerals.

Berberine is a strongly yellow colored quaternary ammonium salt that is found in Berberis plants (e.g. Oregon grape, barberry, and tree turmeric), goldenseal and Chinese goldthread. Berberine is usually found in the roots, rhizomes, stems, and bark. Many studies have shown Berberine has various beneficial effects on the cardiovascular system, such as cholesterol lowering, significant anti-inflammatory activities and reduction of blood glucose levels similar to anti-diabetic medication (Metformin). This was confirmed in a recent meta-analysis of 14 randomized trials involving 1068 participants that concluded that based on the existing evidence reviewed, Berberine has beneficial effects on blood glucose control in the treatment of type 2 diabetic patients and exhibits efficacy comparable with that of conventional oral hypoglycaemics. According to this analysis the antidyslipidemic effect of berberine is highly promising but needs further confirmation. Additionally, it has no serious adverse effects except for a mild to moderate gastrointestinal discomfort (95).

Clinical studies reveal that Berberine reduces total cholesterol, LDL-C and triglyceride levels by 15-18%, 17-21% and 21-36% respectively (14-17). The lipid lowering effect of Berberine is different from and complementary to statins as was shown in animal studies (18). Berberine leads to an increase in LDL receptor by reducing its breakdown, leading to increased uptake of LDL from the blood by the liver. Several successful clinical studies involving in total 2025 participants have been performed to determine the protective effect of a combination of Berberine, Red Yeast Rice and Policosanol to cardiovascular disease risk (19-22). These studies showed significantly lowering of total cholesterol (17-20%), LDL-C (23-31%), triglycerides (13%) and insulin resistance (10%), and a relative increase in HDL levels in patients with elevated cholesterol levels. This result was associated with improved endothelial function (20, 23).

Berberine increases insulin receptor (InsR) expression and improves insulin sensitivity (24). A study in 79 participants with elevated blood glucose levels, showed that Berberine significantly lowered fasting blood glucose and lowered levels of the diabetes marker haemoglobin A(1c) (HBA1c) and insulin. In this study the blood glucose- and HBA1 c-lowering efficacies of Berberine were similar to those of the well-known diabetes medication Metformin. Berberine also lowered fasting blood glucose effectively in 35 chronic hepatitis B and hepatitis C patients with type 2 diabetes or impaired fasting glucose. Liver function was improved greatly in these patients by showing reduction of liver enzymes (16). This was confirmed in another study in which HBA1c decreased from 8.1% to 7.3%. Fasting plasma insulin and insulin resistance index were reduced by 28.1 % and 44.7% (P<.001), respectively. In addition, the glucose disposal rate was increased and thus the insulin resistance decreased after Berberine treatment (14, 15).

In a recent study, Berberine was revealed to markedly reduce the inflammatory cell adhesion to endothelial cells that is induced by oxidized LDL. This inhibitory mechanism of Berberine was associated with suppression of adhesion molecule expression (VCAM-1 and ICAM-1) on endothelial cells. These results indicate that Berberine has a protective role in the early stages of atherosclerosis.

Congestive heart failure is a condition in which the heart's function as a pump is inadequate to deliver oxygen rich blood to the body. A study from 1988 showed in a small number of 12 patients with refractory congestive heart failure that continuous intravenous injection of a high dose Berberine resulted in strongly increased heart function and reduced vascular resistance (96). These results were confirmed in a more recent study from 2003. This study showed Berberine improved quality of life and heart function and decreased mortality in 156 patients with congestive heart failure (97).

The supposed mechanism of action of Berberine is the increased expression of the liver receptors for LDL (LDLr) and insulin (InsR). Besides its up-regulating effect on LDLr and InsR, berberine could also reduce triglycerides by AMP kinase activation and MAPK/ERK pathway blocking (14). The pharmacological actions of Berberine are summarized in a an elaborate review by Cicero and Ertek (98). Due to its peculiar mechanism of action not directly involving the HMGCoA reductase, berberine has been observed to increase the cholesterol-lowering action of both simvastatin and monacolin Kes (17).

Berberine is not considered toxic at the doses used in clinical situations, nor has it been shown to be cytotoxic or mutagenic. The metabolism of Berberine by liver enzymes and the effect of the metabolites on LDLr and InsR expression was characterized by Li et al. (99). In a rat noncompartmental model, Berberine is metabolized by p450 enzyme system in liver, with phase I demethylation and phase II glucuronidation. Berberine has four main metabolites identified in rats: berberrubine, thalifendine, demethyleneberberine, and jatrorrhizine, all of which have glucuronide conjugates (99). The LD50 of berberine HCL is >29586 mg/kg in mice and >15 g/kg in rats (100). In clinical studies performed thus far standard doses below 2 g/day of berberine are well tolerated and adverse reactions are rare and mild.

In the meta analysis of Chinese studies to the efficacy of Berberine by Dong et al. (95), eleven of fourteen trials reported outcomes for adverse effects whereas the rest reported no adverse effects during the Berberine treatment. Three of these reported the incidence of abdominal discomfort, but did not report the group in which abdominal discomfort occurred. Five trials appointed the abdominal discomfort to the Berberine intervention group. In the trial of Cao (101), there were seven incidences of abdominal discomfort like nausea, abdominal distension, and diarrhea. The symptoms were relieved by taking the Berberine postprandial instead of on an empty stomach as initially prescribed. Li and Liu (102) reported a few patients who developed a mild diarrhea caused by the intake of Berberine. In the trial of Wang (103), one incidence of constipation occurred and was relieved after reducing the dose of Berberine to 0.2 g three times a day instead of 0.5 g thrice daily. Zhang et al. (15) also reported mild-to moderate constipation in five participants who took Berberine. Constipation in three participants in the Berberine group was relieved without dose reduction, and two patients with mild constipation reduced Berberine dose to 0.25 g twice daily, resolving the adverse effects. In the trial of Ye (104), the tolerable mild constipation occurred. No severe hypoglycemia was observed in all the included trials in the meta-analysis. In all trials performed thus far no serious adverse events were observed.

On the contrary, high doses equivalent to > 2 g/day of Berberine in isolated cases have been associated with dyspnea, flu-like symptoms and cardiac damage. However, these adverse effects have not been substantiated by clinical trial data. Nevertheless, the most frequent and most studied side effects are those on the gastrointestinal system. In fact, high dose Berberine and derivatives can produce gastric lesions. By using sorbitol and breath hydrogen tests to measure small intestinal transit time, it has been shown that Berberine delays small intestinal transit time. This may account for part of its gastrointestinal and antidiarrheal side effect (105, 106). Another important side effect is that Berberine displaces bilirubin from albumin. Therefore high doses of Berberine should be avoided in jaundiced infants and pregnant woman, as high bilirubin levels can stimulate uterus contraction (107, 108). A composition according to the invention may therefore advantageously contain between 100 and 500 mg of berberine, such as 200 - 400 mg per unit dose, such as 300 mg berberine per unit dose.

The main safety issue of Berberine involves the risk of some pharmacological interaction. Berberine can bind Heparin and therefore has potential interactions with anticoagulant treatment using heparin (109). Berberine can also markedly increase blood levels of cyclosporine A, due to CYP3A4 and P-glycoprotein inhibition in the liver and gut wall. In addition, the increase in gastric emptying time causes increased cyclosporine A bioavailability. This is not a safety issue in healthy individuals, but in renal transplant recipients taking cyclosporine 3 mg/kg twice daily, the coadministration of berberine (0.2 g/day for three times a day for three months) increases the mean cyclosporine A Area under the Curve (AUC) by 34.5% and its mean half-life by 2.7 h (110). Interactions with other drugs that are metabolized by CYP3A4, such as warfarin, lovastatin (Mevacor), clarithromycin (Biaxin), indinavir (Crixivan), sildenafil (Viagra) and triazolam (Halcion) are also expected, but not substantiated by clinical evidence. Although the main mechanism of pharmacological interaction of Berberine involves CYP3A4 and intestinal P-glycoprotein, it was also found to inhibit CYP1A1 in vitro. Therefore Berberine potentially interacts with the relatively rare drugs that are metabolized by this cytochrome isoform (111). Some environmental contaminants such as aryl-hydrocarbons are also metabolized by CYP1A1 and the inhibitory effect of Berberine on CYP1A1 on these compounds has yet to be elucidated.

Finally, Berberine could interact with mitotic inhibitors used in cancer chemotherapy, like Paclitaxel. It was observed that a 24-h Berberine treatment up-regulated the multidrug-resistant transporter (pgp-170) expression in two oral, gastric and colon cancer cell lines. Decreased retention of rhodamine 123 was observed in Berberine-treated cells as compared to vehicle control. Pretreatment of cells with Berberine for 24 h prior to Paclitaxel treatment blocked the Paclitaxel-induced cell cycle responses and morphological changes, resulting in increased viability as compared to that of Paclitaxel-treated cells. These results suggest that Berberine modulates the expression and function of pgp-170 leading to a reduced response to Paclitaxel in digestive track cancer cells (112). Again, no clinical report of a significant pharmacological interaction is yet available.

Overall there are a large number of recent clinical trials supporting the safe use of this nutraceutical up to one year, especially when used at a lipid-lowering dosage < 2 g/day.

Coenzyme Q10 (CoQ10) is found in high concentrations in the mitochondria (power house) of every cell, it is involved in the energy metabolism (electron transport) of cells. As an electron carrier, the CoQ10 molecule is continually going through an oxidation-reduction cycle. As it accepts electrons, it becomes reduced (Ubiquinol) and becomes oxidized again (Ubiquinone) as it gives up electrons. CoQ10 is a powerful antioxidant that prevents oxidative stress caused by our body's energy metabolism. Because nearly all cellular functions depend on an adequate energy supply, CoQ10 deficiency affects all cells, particularly those with high energy consumption, such as the myocardium, neurons and immune cells.

CoQ10 can be synthesised by the body and therefore there is no need for CoQ10 in diets of healthy individuals. There are, however, a number of reported health benefits that can be achieved in populations with either hypertension, heart failure, dementia or people that take statins. Below the specific cardiovascular disease related health benefits of CoQ10 will be summarized.

There is no scientific evidence of a cholesterol lowering effect of coenzyme Q10.

Two meta-analyses one consisting of 3 clinical trials containing a total of 96 participants and one of 12 trials and 362 patients showed that treatment with CoQ10 in subjects with high blood pressure resulted in mean decreases in 11-17 mmHg in systolic and 7-10 mmHg in diastolic blood pressure without significant side effects (30, 31). A third meta-analysis concluded that favourable effects of CoQ10 on heart function (ejection fraction, exercise tolerance, cardiac output, and stroke volume) have been demonstrated in the literature, but further research is needed to recommend CoQ10 as medical therapy (32).

Endothelial dysfunction is one of the main causes of hypertension and has a role in atherosclerotic plaque development. A recent meta-analysis of 5 clinical trials with 194 patients concluded that CoQ10 supplementation is associated with significant improvement in endothelial function (33).

Statin therapy effectively reduces the body's cholesterol production by inhibiting Hydroxymethylglutaryl coenzyme A (HMG-CoA) reductase. However this enzyme is also responsible for the production of CoQ10. Studies have shown that CoQ10 levels are significantly reduced upon statin treatment (132-137). More and more studies demonstrate that CoQ10 supplementation can help to reduce side effects of statin treatment, most importantly myalgia and myopathy (muscle aches, rhabdomyalysis). Several studies have demonstrated reduced side effects of statin treatment after CoQ10 supplementation (34-37). However, some studies report no reduction of myalgia upon CoQ10 supplementation alongside statin treatment (138, 139). It must be noted that in these studies either the levels of myalgia were very low or the bioavailability of the supplement was not tested by measuring coenzyme Q10 blood levels. Thus far, no large clinical trials have confirmed this theory.

Patent EP0383432B1 describes the benefits of CoQ10 supplementation alongside statin treatment. The pharmaceutical use of CoQ10 to reduce statin induced myopathy is described therein.

A recent meta-analysis, consisting of 13 randomized controlled trials of in total 395 participants selected from of 120 potentially relevant studies, suggests that supplementation with CoQ10 may be of benefit in patients with congestive heart failure. However, our findings also suggest that the benefit may be limited to patients with less severe stages of CHF, such as patients with an ejection fraction ≥30% or those with an New York Heart Association (NYHA) functional classification class of II or III. Because of the small number of studies and patients included in this meta-analysis, the results should be interpreted with caution. Although there is a possible benefit of supplementation with CoQ10, additional larger studies are warranted and should examine whether there is an effect when this supplementation is added to the current standard of therapy for CH F or whether there is a dose-response effect between the stage of CHF at baseline and the dose of CoQ10 required for an improvement to be seen (140).

CoQ10 undergoes biotransformation in the liver and is eliminated primarily through the biliary tract, so it can accumulate in patients with hepatic impairment or bile duct obstruction (141). There are few reported serious adverse effects of CoQ10. Side effects are typically mild and brief. Taking 100 mg a day or more of CoQ10 has caused mild insomnia in some people. Research has detected elevated levels of liver enzymes in people taking doses of 300 mg per day for long periods of time. Liver toxicity has not been reported. Other reported side effects include rashes, skin itching, nausea, vomiting, stomach upset, diarrhea, loss of appetite, upper abdominal pain, dizziness, sensitivity to light, irritability, headache, heartburn, fatigue or flu-like symptoms (142).

The acceptable daily intake (ADI) is 12mg/kg/day, calculated from the no-observed-adverse-effect level (NOAEL) of 1200 mg/kg/day derived from a 52-week chronic toxicity study in rats, this corresponds with 720 mg/day for a person weighing 60 kg. Risk assessment for CoQ10 based on various clinical trial data indicates that the observed safety level (OSL) for CoQ10 is 1200 mg/day/person.

CoQ10 does have some interactions with existing drugs. Since CoQ10 is able to decrease blood pressure caution is advised for individuals with low blood pressure or taking blood pressure medications. In addition, CoQ10 can reduce the body's response to warfarin (Coumadin) by acting as a vitamin K substitute, leading to altered dosing requirements for warfarin (143). Coenzyme Q10 may improve beta-cell function and enhance insulin sensitivity, which may reduce insulin requirements for diabetic patients (144, 145). One study indicated that Thyroid hormone levels are associated to CoQ10 levels and might be altered after CoQ10 supplementation (146).

A composition according to the invention may therefore advantageously contain between 1 and 200 mg of coenzyme Q10, such as 100 mg coenzyme Q10 per unit dose.

Ginseng is a well-known medical herb with a long list of acclaimed health benefits. Ginsenosides, known as ginseng saponins, are the major components of ginseng. Ginseng also contains several valuable nonsaponin components, including essential oils, antioxidants, polyacetylenic alcohols, peptides, amino acids, polysaccharides, and vitamins. A very elaborate overview of ginseng's medical applications is given by Jae Joon Wee et al. (147).

One of the most important acclaimed health effects is protection of neurons, which leads to improved learning and memory and prevention of Alzheimer's disease. However these effects have not yet been confirmed in human trials. In case-control studies, the chance of cancer of the lip, oral cavity and pharynx, larynx, lung, oesophagus, stomach, liver, pancreas, ovary, and colorectum were significantly reduced after using ginseng supplements (148, 149). In addition ginseng has been suggested to be effective in improving psychomotor function, cognitive functioning (150, 151), erectile function (152), and pulmonary disease (153). The following in depth information will be limited to ginseng's acclaimed health benefits for cardiovascular disease prevention.

Vasodilation (relaxing of the vessel wall), ischemic heart disease and angina pectoris (heart related chest pain).

Ginseng-based medicines and nitrates are commonly used in treating ischemic heart disease (IHD) and angina pectoris in China. Hundreds of randomized controlled trials reported in Chinese language claimed that ginseng-based medicines can relieve the symptoms of IHD by vasodilation of the coronary arteries. A systematic review containing eighteen randomized placebo controlled trials with 1549 participants investigating ginseng versus nitrates in treating IHD, demonstrated evidence that ginseng is more effective than nitrates for treating angina pectoris (38). These are highly promising results as nitrates are an accepted treatment in western countries for angina pectoris. This effect is attributed to the vasodilator function of ginseng, which has been shown in several animal studies and two small clinical trials (40, 154). The vasodilator function might also help to reduce blood pressure, but very limited evidence is available and seemingly high doses of ginseng are needed (39-41).

A very recent randomized controlled trial to the antioxidant activity of ginseng showed strong antioxidant effects in humans (42). Plasma superoxide dismutase (SOD), plasma glutathione peroxidase (GPx) and catalase activity were significantly higher after 8-week ginseng treatment. Furthermore, the DNA tail length and tail moment, both measures for DNA damage by oxidative stress were significantly reduced. More importantly, plasma levels of oxidized LDL, a major constituent to cardiovascular disease development, were reduced as well. Unfortunately study duration was short and sample size limited, making it hard to translate the study outcome to the general population.

The anti-inflammatory effects of Ginseng appear to be limited to men. A recent epidemiological study in 9,947 subjects showed that taking ginseng supplements is associated with a reduction of the inflammation risk marker for cardiovascular disease HS-CRP by 16% in men. While ginseng supplements had no effect in women (43). It is interesting to note that in a large 18 year long cohort study of 6282 subjects, Yi et al. reported an inverse association between ginseng use and total mortality, an association which was similarly limited to men (44).

Several human randomized controlled clinical trials investigating the effect of red ginseng on type 2 diabetes have been performed but remain inconclusive (155). Clinical studies with small sample sizes (10-16 patients) have reported that American ginseng lowers postprandial blood glucose in diabetic and non-diabetic patients (156, 157). Animal studies clearly show protective effects of ginseng and its components against insulin resistance and diabetes. However, confirmation in human trials is lacking (155). Therefore, more studies are required to confirm that ginseng administration decreases the dietary glycaemic index and is preventive of insulin resistance.

A recent study in postmenopausal women showed significant improvements (Kupperman index and menopause rating scale) and reduction of total cholesterol and LDL after taking ginseng (158). However, no changes in cholesterol or triglycerides were detected in a study in hypertensive patients (41).

The use of Panax ginseng is generally considered safe. No reports of serious adverse effects are available. A recent systematic review of the efficacy and safety of Panax Ginseng including 57 clinical trials reported the following side effects (159). Thirty of the 57 trials reported the presence or absence of adverse events: 16 reported some side effects, whereas 14 found no side effects during the trials. The most common side effect is trouble sleeping (insomnia). Less commonly, people experience menstrual problems, breast pain, increased heart rate, high or low blood pressure, headache, loss of appetite, diarrhea, itching, rash, dizziness, mood changes, vaginal bleeding, abdominal pain, dyspepsia, hot flash and epistaxis. Most are associated with higher doses of Panax Ginseng of 500 mg per day. The 27 other trials did not address the topic.

There is not enough scientific evidence to support the safe use of Panax Ginseng during pregnancy or breastfeeding. There are some indications that Panax Ginsing might interfere with blood coagulation, therefore use in combination with anticoagulants requires careful monitoring of hemostasis.

A composition according to the invention may therefore advantageously contain between 1 and 40 mg of ginseng, such as 20 mg per unit dose.

Flavonoids are the most important plant pigments responsible for flower and fruit coloration producing yellow or red/blue pigmentation. The flavonoid subgroup anthocyanidins (anthocyanins) for example are the components that give berries (Blue-, cran- and bill-berries) their specific dark blue color. Other fruits and vegetables with red or orange colors also contain anthocyanins (166). Proanthocyanidins are the principal substances in red wine that are linked to a reduced risk of coronary heart disease and to lower overall mortality (167). Proanthocyanidins, refer to a different class of flavonoids, called flavanols, in which occur PCOs (proanthocyanidin oligomers) or the more common dietary supplement ingredient OPCs (oligomeric proanthocyanidins) or in more complex form the well-known tannins.

Several mechanisms of action of flavonoids in general have been described (168). Strong anti-oxidant induction properties. Oxidative stress has been implicated in the development of a number of conditions including cancer, arthritic disorders and cardiovascular disease. Stabilization of collagen fibers and promotion of collagen biosynthesis and decreased permeability and fragility of capillaries has been attributed to flavonoids. Also, flavonoids have been held responsible for inhibition of blood clotting (platelet aggregation), reduction of inflammation by prevention of the release and synthesis of pro-inflammatory compounds (e.g. histamine, prostaglandins, and leukotrienes) and lower blood glucose levels

Oxidative stress caused by free radicals is one of the driving forces behind ageing and the development of cardiovascular disease, dementia and cancer. The importance and protective effect of daily intake of antioxidants is well known and has been scientifically proven. Interestingly, most of the antioxidant function of the human body is not derived from the consumption of dietary antioxidants. Dietary antioxidants undeniably contribute to the body's antioxidant function, but the most potent antioxidants are produced by the body itself. The increased antioxidant function of the human body after consumption of fruits and vegetables is mostly due to an induction of the body's own antioxidant production by the flavonoids in them (169). Recently researchers discovered that the metabolites derived from digestion of flavonoids are light toxic substances. Due to their toxicity these substances activate the natural antioxidants and defense of the human body, such as the very strong antioxidant uric acid. These are far more efficient than dietary antioxidants and therefore offer highly valuable protection against oxidative stress (170-172).

Flavonoids have emerged as potential candidates to protect against cardiovascular disease. Many recent human studies suggest that the consumption of flavonoid rich food (Strawberries, Blueberries, Red Wine) decreases the risk of cardiovascular disease mortality. Relatively low-dose anthocyanin interventions (mostly in the form of pomegranate juice) with patients clinically diagnosed with vascular diseases have been associated with significant reductions in heart ischemia (173), intima-media thickness (thickness of the arterial wall), blood pressure, LDL oxidation (174), lipid levels (175), and inflammatory status (176). This last study was a double-blind, placebo-controlled, parallel trial in forty-four patients who survived myocardial infraction and have received statin therapy for at least 6 months. Chokeberry flavonoid extract (Aronia melanocarpa E, containing 25% anthocyanins, 50% polymeric proanthocyanidins and 9% phenolic acids) supplementation for a period of 6 weeks significantly reduced serum 8-isoprostans by 38% and oxLDL levels by 29%, as well as the inflammatory markers hsCRP by 23% and MCP-1 levels by 29%. In addition, significant increase in the protective adiponectin levels and reduction in systolic and diastolic blood pressure by a mean average of 11 and 7.2 mmHg, respectively were found. A recent study by Karlsen et al. (177) showed significant improvement of plasma risk biomarkers after supplementation with anthocyanins. Several pro-inflammatory cytokines and chemokines were reduced in the plasma of healthy men and women after supplementation with anthocyanins (178, 179). In view of the fact that flavonoids reduce the severity of inflammation, regardless of statins, they are a highly interesting supplementation alongside statin treatment (176).

A large number of cohort studies assessed the association between flavonoid intake and cardiovascular disease incidence and mortality. A recent review of existing studies revealed that associations are present between flavonoid intake and cardiovascular disease risk and mortality, but that results are not consistent (180). A meta-analysis of 6 cohort studies revealed that coronary heart disease risk decreased significantly with increased flavonoid intake. A large 16-year follow-up study in 34,489 post-menopausal women without cardiovascular disease showed that the intake of 0.2mg/day of the flavonoid classes, flavones and anthocyanins was associated with a decreased risk of cardiovascular disease (181). In another very recent study, this protective effect was substantiated (182). In 1999, a total of 38,180 men and 60,289 women, with a mean age of 70 and 69 y respectively were included in the Cancer Prevention Study II Nutrition Cohort. During 7 years of follow-up, 1589 cardiovascular disease related deaths in men and 1182 cardiovascular disease related deaths in women occurred. Men and women with total flavonoid intakes in the top (compared with the bottom) quintile had an 18% lower risk of fatal cardiovascular disease. Five flavonoid classes-anthocyanidins, flavan-3-ols, flavones, flavonols, and proanthocyanidins-were individually associated with lower risk of fatal cardiovascular disease (183). Several other large cohort studies showed that daily moderate red wine consumption resulted in reduced CVD risk (184-186). These effects are often attributed to the antioxidant properties of flavonoids.

A composition according to the invention may therefore advantageously contain between 1 and 500 mg of flavonoids, such as 200 - 400 mg per unit dose, such as 300 mg flavonoids per unit dose.

Vitamin K2 (menachinone, menaquinone, MK-4, MK-7, MK-9) has an important role in bone mineralization, arterial calcification, calcification of the heart valves tissue repair and according to a recent study also in insulin response. This is confirmed by recent studies showing that anti-coagulant medication (coumarin derivates) which inhibit vitamin K function lead to an increase in osteoporosis and artery calcification (195-197).

Vitamin K2 helps to reduce calcification of the arteries and heart valves. Two large Dutch studies have investigated this phenomenon. A study in 4,807 elder men and women, published in 2004 in the Journal of Nutrition showed that in the group with a high dietary intake of vitamin K2 had 57% less myocardial infarctions, 52% less calcification of the aorta and 26% less overall mortality compared to the group with al low dietary intake of vitamin K2 (63). After 8 years and 480 cardiovascular events, the cohort study Prospect EPIC in 16,057 women aged 49-70, showed that each increase in dietary intake of vitamin K2 by 10 microgram resulted in a risk reduction of 9% (64). Several other well-designed clinical trials showed that vitamin K2 helps to reduce osteoporosis (64-69).

At Geneva's Vitafoods 2012, Dr. Cees Vermeer, Principal Investigator at VitaK laboratory at Maastricht University, has presented data showing significant benefits for improved bone strength and prevention of cardiovascular aging with daily supplementation of MenaQ7. In the study, 244 healthy postmenopausal women received for 3 years daily 180 µg K2 from MenaQ7 or a placebo. The clinical measurements included bone mineral density, bone strength, vascular characteristics by ultrasound and pulse-wave velocity (PWV), the latter evaluating age-related stiffening of blood vessels. MenaQ7 supplementation provided a statistically significant protection of the most vulnerable bone structures i.e. vertebrae and the hip. The MenaQ7 trial showed substantial benefits in preventing age-related stiffening of arteries resulting in increase of a measure for arterial stiffness (Pulse Wave Velocity) in the placebo group, but not in the MenaQ7-group. Most remarkably, MenaQ7 not only prevented stiffening, it also resulted in a statistically significant improvement of vascular elasticity both measured with ultrasound techniques and Pulse Wave Velocity. http://www.cisionwire.com/nattopharma-asa/r/menaq7--3-year-human-study-with-vitamin-k2-demonstrates-that-menaq7--significantly-improves-bone-str,c9262978

There are indications that vitamin K2 helps to improve insulin response. However, this is not yet substantiated by large clinical trials (198).

A composition according to the invention may therefore also advantageously contain between 20 and 300 microgram of vitamin K2, such as 180 microgram per unit dose.

A composition according to the invention may also advantageously contain between 50 and 400 microgram of folic acid or B vitamins, preferably each at about 200 micrograms per dose unit.

A composition according to the invention may also advantageously contain between 0,5 and 4 mg of biotin (vitamin H), preferably each at about 2 mg per dose unit.

A composition according to the invention may also advantageously contain minerals, such as Calcium, Magnesium, Selenium, Zinc, Iodine or Chromium.

The invention also relates to the medical use of the compositions as described herein. In other terms, the invention relates to a method of treating a disease by administering a composition as described herein to a subject in need of such a treatment.

More in particular, the invention relates to a composition as described herein for use in the treatment or prevention of a disease or for reducing foam cell formation and progression.

The invention also relates to a composition for use as described above wherein the disease is selected from the group consisting of heart disease, cardiovascular disease, hypercholesterolaemia, hypertriglyceridaemia, hyperlipidemia, type II diabetes, elevated blood pressure, hypertension, pre-diabetes, myocardial infarction, stroke, heart failure, chronic heart failure and congestive heart failure.

Table 1 lists the active ingredients of the compositions according to the invention together with their preferred daily dose and ranges for administration.

**Table 1 list of active ingredients**

| **Active ingredient** | **Biological effect** | **Amount per unit dose (range)** |
|---|---|---|
| *Monacolin K* | • Natural Statin (HMG CoA inhibitor) Monacolin K | 10 mg (2 - 40) |
| | • LDL cholesterol lowering of 7-25% | |
| | • Relative increase of HDL up to 17% | |
| | • Lowering of triglyceride blood levels by 10-44% | |
| Lycopene | • LDL cholesterol lowering of 10% | 25 mg (1 - 100) |
| | • Systolic blood pressure lowering by 5.6-9.5 mmHg | |
| Vitamin D3 | • Overall CVD risk reduction | 25 microgram (1 - 100) |
| | • Reduction left ventricular hypertrophy | |
| | • Reduction arterial stiffness | |
| Berberine | • Upregulation LDL-receptor | 300 mg (100-500) |
| | • LDL cholesterol lowering of 17-25% | |
| | • Lowering of triglyceride blood levels by 21-36% | |
| | • Blood glucose- and HBA1c-lowering efficacies similar to metformin and rosiglitazone. | |
| Coenzyme Q10 | • Systolic blood pressure lowering by 11-17mmHg | 100 mg (1 - 200) |
| | • Diastolic blood pressure lowering by 7-10 mmHg | |
| Panax Ginseng | • Vasodilation | 20 mg (1 - 40) |
| | • Anti-oxidant | |
| | • Immune function | |
| | • Lowering oxLDL serum levels | |
| Flavonoids | • Vasodilation | 200 mg (1 - 500) |
| | • Anti-oxidant | |
| | • Immune function | |
| | • Lowering oxLDL serum levels | |
| Vitamin K2 | • Reduction of arterial calcification | 180 microgram (20 - 300) |
| | • Reduction of osteoporosis | |
| Folic acid + B vitamins | • | 200 microgram (50 - 400) |
| Biotin | • | 2 mg (0,5 - 4) |
| Total weight of preferred embodiment | • | Less than 700 mg |

### Legend to the figures

Figure 1: Bar diagram showing the property of vitamin D3, monacolin K and lycopene to inhibit the uptake of oxLDL by pma-stimulated human THP-1 cells, individually and in synergy with each other.

### Examples

### Example 1 Model system

THP-1 cells (P7) were plated in a 96-wells suspension culture plate at a density of 1*10^6 cells/ml whereby 1*10^5 cells were plated. Cells were cultured in IMDM phenol-red free medium (Invitrogen, Eugene, OR, USA) enriched for 10% fetal calf serum (FCS), 100 U/ml penicillin, 100 µg/ml streptomycin and 2 mM L-glutamin. Monocytes were differentiated with 50ng/ml phorbol 12-myristate 13-acetate (PMA) (Sigma Aldrich, St. Louis, MO USA) for 48h where after medium was replaced. After differentiation, macrophages were cultured in the presence or absence of different compounds for 24h (Table 2). After pre-treatment, cells were exposed to 10µg/ml Dillabeled oxidized lipoprotein (oxLDL) (Biotrend Chemikalien GmbH, Cologne, Germany) for another 24h at 37°C to enable foam cell formation in the absence of presence of the different compounds (Figure 1). After this, cells were washed with PBS (-/-) (PAA, Pitscataway, NJ, USA) and detached by Lidocaine hydrochloride (4mg/ml) EDTA (10mM) solution (Sigma Aldrich, St. Louis, MO USA)/ (Invitrogen, Eugene, OR, USA). Cells were washed two more times with FACS buffer (PBS containing 0,5% BSA and 0.2mM EDTA) and transferred to a 96 U-shaped plate for FACS analysis. Analysis was performed on the FACS Canto II HTS and 1,1'-dioctadecyl-3,3,3'3'-tetramethylindocyanide percholorate (Dil)-labeled oxLDL uptake was measured in PE-A channel.

**Table 2 Active ingredients used in this study.**

| ***Compound*** | ***Concentration*** |
|---|---|
| Vitamin D3 | 100nM |
| Lycopene | 0.5uM |
| Monacolin K | 10uM |
| Lycopene + Monacolin K | 0.5uM/ 10uM |
| Vitamin D3 + Monacolin K | 100nM/ 10uM |
| Lycopene + Vitamin D3 | 0.5uM/ 10uM |
| Monacolin K + Lycopene + Vitamin D3 | 10uM/ 0.5uM/ 100nM |

### Example 2: Inhibition of oxLDL uptake by macrophages under the influence of 3 active ingredients.

Macrophages were exposed to vitamin D3, lycopene and/or monacolin K and the inhibitory effect of oxLDL uptake of these three active ingredients was measured. The results of 6 independent measurements are shown in table 3. The inhibitory potentiol of each compound and composition was expressed as the difference between the measurements obtained with and without the compound or composition. The inhibitory potential of the compounds and compositions is shown in figure 1.

**Table 3; Inhibitory effect of three compounds on the uptake of oxLDL by macrophages.**

| **Active ingredient** | **oxLDL uptake [Median Fluorescence intensity]** | **Average** |
|---|---|---|
| Control | 12600 | |
| Control | 13300 | |
| Control | 12500 | |
| Control | 11900 | |
| Control | 13800 | |
| Control | 11600 | 12617 |
| VitD3 (100nM) 1 | 13100 | |
| VitD3 (100nM) 2 | 13200 | |
| VitD3 (100nM) 3 | 13100 | |
| VitD3 (100nM) 4 | 12600 | |
| VitD3 (100nM) 5 | 10900 | |
| VitD3 (100nM) 6 | 10900 | 12300 |
| Lycopene (0'5uM) 1 | 9832 | |
| Lycopene (0'5uM) 2 | 11500 | |
| Lycopene (0'5uM) 3 | 12200 | |
| Lycopene (0'5uM) 4 | 11900 | |
| Lycopene (0'5uM) 5 | 11300 | |
| Lycopene (0'5uM) 6 | 11700 | 11405 |
| Monacolin K (10uM) 1 | 14300 | |
| Monacolin K (10uM) 2 | 11900 | |
| Monacolin K (10uM) 3 | 11900 | |
| Monacolin K (10uM) 4 | 11700 | |
| Monacolin K (10uM) 5 | 11800 | |
| Monacolin K (10uM) 6 | 14400 | 12667 |
| Monacolin K (10uM) + Lycopene (0'5uM) 1 | 11800 | |
| Monacolin K (10uM) + Lycopene (0'5uM) 2 | 12000 | |
| Monacolin K (10uM) + Lycopene (0'5uM) 3 | 10800 | |
| Monacolin K (10uM) + Lycopene (0'5uM) 4 | 13800 | |
| Monacolin K (10uM) + Lycopene (0'5uM) 5 | 12600 | |
| Monacolin K (10uM) + Lycopene (0'5uM) 6 | 11400 | 12067 |
| Monacolin K (10uM) + VitD3 (100nM) 1 | 12100 | |
| Monacolin K (10uM) + VitD3 (100nM) 2 | 12400 | |
| Monacolin K (10uM) + VitD3 (100nM) 3 | 11600 | |
| Monacolin K (10uM) + VitD3 (100nM) 4 | 12800 | |
| Monacolin K (10uM) + VitD3 (100nM) 5 | 10500 | |
| Monacolin K (10uM) + VitD3 (100nM) 6 | 10200 | 11600 |
| Lycopene (0'5uM) + VitD3 (100nM) 1 | 14500 | |
| Lycopene (0'5uM) + VitD3 (100nM) 2 | 11700 | |
| Lycopene (0'5uM) + VitD3 (100nM) 3 | 12200 | |
| Lycopene (0'5uM) + VitD3 (100nM) 4 | 11900 | |
| Lycopene (0'5uM) + VitD3 (100nM) 5 | 11000 | |
| Lycopene (0'5uM) + VitD3 (100nM) 6 | 9640 | 11823 |
| Monacolin K (10uM) + Lycopene (0'5uM) + VitD3 (100nM) 1 | 9876 | |
| Monacolin K (10uM) + Lycopene (0'5uM) + VitD3 (100nM) 2 | 9261 | |
| Monacolin K (10uM) + Lycopene (0'5uM) + VitD3 (100nM) 3 | 8222 | |
| Monacolin K (10uM) + Lycopene (0'5uM) + VitD3 (100nM) 4 | 9421 | |
| Monacolin K (10uM) + Lycopene (0'5uM) + VitD3 (100nM) 5 | 10300 | |
| Monacolin K (10uM) + Lycopene (0'5uM) + VitD3 (100nM) 6 | 10500 | 9597 |

## Claims

1. Composition for oral administration having a beneficial effect on the cardiovascular system, comprising as active ingredients a mixture of monacolin K, lycopene and vitamin D3.

2. Composition according to claim 1 further comprising an active ingredient selected from the group consisting of Berberine, coenzyme Q10, panax ginseng, flavonoids, vitamin K2, folic acid, vitamin B, biotin and minerals.

3. Composition according to claim 2 wherein the minerals are Calcium, Magnesium, Selenium, Zinc, Iodine or Chromium.

4. Composition according to any one of claims 1 - 3 in the form of a unit dose.

5. Composition according to any one of claims 1 - 4 comprising between 2 and 40 mg of monacolin K per unit dose, preferably about 10 mg per unit dose.

6. Composition according to any one of claims 1 - 5 comprising between1 and 100 mg of lycopene per unit dose, preferably about 25 mg per unit dose.

7. Composition according to any one of claims 1 - 6 comprising between 1 and 100 microgram of vitamin D3 per unit dose, preferably about 25 microgram per unit dose.

8. Composition according to any one of claims 1 - 7 wherein the unit dose comprises a pill, sachet, capsule, bulk powder container, tablet or equivalent suited for oral administration to a subject.

9. Composition according to any one of claims 1 - 8 for use in the treatment or prevention of a disease or for reducing foam cell formation and progression.

10. Composition for use according to claim 9 wherein the disease is selected from the group consisting of heart disease, cardiovascular disease, hypercholesterolaemia, hypertriglyceridaemia, hyperlipidemia, type II diabetes, elevated blood pressure, hypertension, pre-diabetes, myocardial infarction, stroke, heart failure, chronic heart failure and congestive heart failure.
